# EUROPEAN PATENT SPECIFICATION

(11) **EP 2 392 329 B1**
(45) Date of publication and mention of the grant of the patent: **03.10.2012**
(21) Application number: 11168376.9
(22) Date of filing: 01.06.2011
(51) Int. Cl.: A61K 31/454, A61K 38/06, A61K 9/00, A61P 35/00

(54) **Compositions for use in treating myelodysplastic syndrome**
Zusammensetzungen zur Verwendung bei der Behandlung des myelodysplastischen Syndroms
Compositions à utiliser dans le traitement d'un syndrome myélodysplasique

(30) Priority: 07.06.2010 US 352374 P; 16.05.2011 US 108756
(43) Date of publication of application: 07.12.2011
(73) Proprietor: Telik, Inc., Palo Alto, CA 94304 (US)
(72) Inventor: Brown, Gail L., Palo Alto, CA California 94304 (US); Meng, Lixin, Palo Alto, CA California 94304 (US)
(74) Representative: Gibson, Mark

(56) References cited:
- RAZA AZRA ET AL: "Phase 2 Randomized Multicenter Study of Extended Dosing Schedules of Oral Ezatiostat HCl (Telintra), a Glutathione Analog Prodrug GSTP1-1 Inhibitor, In Low to Intermediate-1 Risk Myelodysplastic Syndrome (MDS)", BLOOD, AMERICAN SOCIETY OF HEMATOLOGY, US, vol. 116, no. 21, 1 November 2010 (2010-11-01), page 1198, XP009150424, ISSN: 0006-4971
- ANONYMOUS: "Telik report phase II data on ezatiostat in MDS", INTERNET CITATION, [Online] 9 June 2010 (2010-06-09), page 1, XP002651700, Retrieved from the Internet: URL:https://integrity.thomson-pharma.com/i ntegrity/xmlxsl/pk_ref_list.xml _show_ficha_ref?p_ref_id=1513842> [retrieved on 2011-07-20]
- ANONYMOUS: "Telik initiates phase I trial of ezatiostat in patients with myelodysplastic syndrome", INTERNET CITATION, [Online] 27 January 2010 (2010-01-27), page 1, XP002651699, Retrieved from the Internet: URL:https://integrity.thomson-pharma.com/i ntegrity/xmlxsl/pk_ref_list.xml _show_ficha_ref?p_ref_id=1444034> [retrieved on 2011-07-20]
- anonymous: "Dose-Ranging Study of Telintra® Tablets + Revlimid® in Patients With Non-Deletion (5q) Low to Intermediate-1 Risk Myelodysplastic Syndrome (MDS)", , 28 April 2010 (2010-04-28), XP002660202, Retrieved from the Internet: URL:http://clinicaltrials.gov/archive/NCT0 1062152/2010_04_28 [retrieved on 2011-09-26]

## Description

### Field of the Invention

The invention relates to compositions for use in treating myelodysplastic syndrome.

### Background of the Invention

Myelodysplastic syndrome(s) (MDS) refers to a heterogeneous group of clonal hematopoietic stem cell disorders characterized by ineffective hematopoiesis (blood cell production) involving one or more cell lineages (red blood cells, white blood cells or platelets) and a variable risk of transformation to acute myeloid leukemia (AML). This syndrome becomes more common with age. It is estimated that MDS affects approximately 300,000 people worldwide. According to the American Cancer Society, 10,000 to 20,000 new cases of MDS are diagnosed each year in the United States alone. Survival rates using current therapy range from 6 months to 6 years with patients often requiring blood transfusions to manage their disease.

Currently, there are three approved drugs for treating MDS by the U.S. Food and Drug Administration (FDA). Lenalidomide is indicated for the treatment of transfusion dependent MDS patients with del(5q) and lower risk disease while azacytidine and decitabine are approved for all categories. With the exception of del(5q) patients, the response rate is approximately 50%, highlighting the need for clinical trials of new agents.

Ezatiostat and its salts are disclosed in US Patent No. 5,763,570. Ezatiostat has the IUPAC chemical name of ethyl (2*S*)-2-amino-5-[[(2*R*)-3-benzylsulfanyl-1-[[(1*R*)-2-ethoxy-2-oxo-1-phenylethyl]amino]-1-oxopropan-2-yl]amino]-5-oxopentanoate.

One example of a salt of ezatiostat is the hydrochloride salt, ezatiostat hydrochloride (USAN), which has the molecular weight of 566.1, the trademark of Telintra®, and the CAS registry number of 286942-97-0. U.S. Patent Application No. 13/041,136, filed March 4, 2011, describes ansolvate and polymorphs of ezatiostat hydrochloride.

Ezatiostat hydrochloride has been evaluated for the treatment of MDS, in a Phase I-IIa study using a liposomal formulation (US Patent No. 7,029,695), as reported at the 2005 Annual Meeting of the American Society for Hematology (Abstract #2250) and by Raza et al. in Journal of Hematology & Oncology, 2:20 (published online on 13 May 2009); and in a Phase I study using a tablet formulation, as reported at the 2007 Annual Meeting of the American Society for Hematology (Abstract #1454) and by Raza et al. in Blood, 113:6533-6540 (prepublished online on 27 April 2009), and in a single patient case report by Quddus et al. in Journal of Hematology & Oncology, 3:16 (published online on 23 April 2010).

### Summary of the Invention

The invention relates to the discovery of the problem that patients with a myelodysplastic syndrome who have been treated with a DNA methyltransferase inhibitor did not respond to treatment with ezatiostat hydrochloride. The invention is further based on the surprising discovery that the response rate to ezatiostat hydrochloride is increased in patients who had lenalidomide treatment prior to administration of ezatiostat hydrochloride.

Accordingly, in one aspect, the invention is directed to a composition comprising ezatiostat or a salt thereof for use in treating a myelodysplastic syndrome in a patient who has been treated with a DNA methyltransferase inhibitor, wherein lenalidomide is administered to said patient prior to and/or concurrently with administration of said composition.

In some embodiments, ezatiostat or a salt thereof is administered by a dosing regimen described in U.S. Patent Application No. 13/108,752, titled "COMPOSITIONS AND METHODS FOR TREATING MYELODYSPLASTIC SYNDROME," filed May 16, 2011 under the Attorney Docket No. 056274-4301, and which claims priority to US Provisional Application No. 61/352,371, filed on June 7, 2010. For example, ezatiostat or a salt thereof may be administered in cycles of 2 gram/day orally for 3 weeks on/1 week off, or cycles of 3 gram/day orally for 2 weeks on/1 week off. Equivalent ezatiostat doses for ezatiostat itself or other ezatiostat salts, or for other routes of administration may also be used.

In one embodiment, ezatiostat or a salt thereof can be administered as a tablet formulation. Such a tablet formulation is disclosed in U.S. Patent Application No. 13/075,116, filed March 29, 2011, titled "TABLET FORMULATION OF EZATIOSTAT".

In another aspect, the invention provides a composition for treating a myelodysplastic syndrome in a patient who has been treated with a DNA methyltransferase inhibitor, which composition comprises lenalidomide, ezatiostat or a salt thereof, and optionally a pharmaceutically acceptable excipient.

In still another aspect, the invention provides a kit of parts for treating a myelodysplastic syndrome in a patient who has been treated with a DNA methyltransferase inhibitor, which kit comprises a first composition comprising lenalidomide and a second composition comprising ezatiostat or a salt thereof.

These and other embodiments of the invention are further described in the text that follows.

### Detailed Description of the Invention

Prior to describing the invention in greater detail, the following terms will first be defined.

It is to be understood that the invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

It must be noted that as used herein and in the appended claims, the singular forms "a", "an", and "the" include plural referents unless the context clearly dictates otherwise. Thus, for example, reference to "a DNA methyltransferase inhibitor" includes a plurality of DNA methyltransferase inhibitors (DMTIs).

### 1. Definitions

Unless defined otherwise, all technical and scientific terms used herein have the same meaning as commonly understood by one of ordinary skill in the art to which the invention belongs. As used herein the following terms have the following meanings.

The term "comprising" or "comprises" means that the compositions and uses include the recited elements, but do not exclude others. "Consisting essentially of" when used to define compositions and uses, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" means excluding more than trace elements of other ingredients and substantial method steps. Embodiments defined by each of these transition terms are within the scope of the invention.

The term "about" when used before a numerical designation, e.g., temperature, time, amount, and concentration, including range, indicates approximations which may vary by ( + ) or ( - ) 15 %, 10 %, 5 % or 1 %.

"Lenalidomide" (Revlimid®, also known as Revamid in the UK) is an immunomodulatory agent with antiangiogenic and antineoplastic properties. It has the chemical name of 3-(4-amino-1-oxoisoindolin-2-yl)piperidine-2,6-dione or 1-oxo-2-(2,6-dioxopiperidin-3-yl)-4-aminoisoindoline or 3-(7-amino-3-oxo-1H-isoindol-2-yl)piperidine-2,6-dione, and CAS registry number of 191732-72-6. Lenalidomide is indicated for the treatment of patients with transfusion-dependent due to low- or intermediate-1 risk MDS associated with a deletion 5q cytogenetic abnormality. Lenalidomide is available in 5 milligram (mg), 10 mg, 15 mg and 25 mg capsules for oral administration.

The term "therapeutically effective amount" refers to the amount of either lenalidomide or ezatiostat or a salt thereof that is an amount sufficient to effect treatment, as defined herein, when administered to a subject in need of such treatment. In one embodiment, the therapeutically effective amount will be up to 3.5 grams (g) of ezatiostat or a salt thereof administered per day. Preferably, ezatiostat or a salt thereof is administered in an amount of 2 grams per day and, more preferably, is administered twice a day in equal 1 gram doses. Such a therapeutically effective amount is particularly relevant when the treatment regimen is for 3 weeks of administration of ezatiostat or a salt thereof followed by a week of no administration of the drug. In another embodiment, the therapeutically effective amount will be up to 3 grams of ezatiostat or a salt thereof administered in a single dose, or in 2 equal daily doses of up to 1.5 grams. Such a therapeutically effective amount is particularly relevant when the treatment regimen is for 2 weeks of administration of ezatiostat or a salt thereof followed by a week of no administration of the drug. Preferably, the dosing regimen employs 2 grams of ezatiostat or a salt thereof administered in an amount of 1 gram doses twice a day either under continuous administration or with administration for 3 weeks followed by a week of no administration of the drug.

In a preferred embodiment, the therapeutically effective amount will provide efficacious results in at least about 10 % of the treated population, and preferably at least about 15 %.

As used herein, the term "treatment" or "treating" means any treatment of MDS in a patient which produces one or more of the following:
- inhibiting the MDS, that is, arresting or suppressing the development of symptoms (e.g., need for blood transfusion, abnormal blood count, and the like); and/or
- relieving the MDS, that is, causing the regression of symptoms.

As used herein, the term "patient" refers to mammals and includes humans and non-human mammals.

### 2. Composition

In one aspect, the invention is directed to a composition for use in treating a myelodysplastic syndrome (MDS) in an MDS patient who has been treated with a DNA methyltransferase inhibitor (DMTI).

DMTIs, also known as demethylating agents, are a class of agents that inhibit methylation of DNA through inhibition of the DNA methyltransferase activity. Methylation of DNA is a major mechanism that regulates gene expression in cells. When there is an increase in DNA methylation this can result in the blockage of the activity of "suppressor genes" that regulate cell division and growth.

Examples of DMTIs include analogs of the nucleoside deoxycitidine, such as azacitidine (5-azacytidine), decitabine (5-aza-2'-deoxycytidine), 1-β-d-arabinofuranosyl-5-azacytosine and dihydro-5-azacytidine; and antisense oligodeoxynucleotide, such as MG98 (by MGI Pharma, Inc.), which is directed against the 3'-untranslated region of the DNA methyltransferase-1 enzyme mRNA and is now under clinical study. Other DMTIs are described in Lyko F and Brown R., J. Natl. Cancer Inst., 2005; 97(20):1498-506.

5-Azacytidine (or azacitidine (INN), Vidaza®, CAS registry number 320-67-2), is an analogue of cytidine and has the formula:

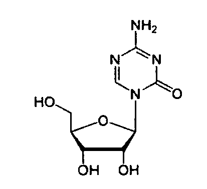

Decitabine (or 5-aza-2'-deoxycytidine, Dacogen®, CAS registry number of 2353-33-5), is a cytosine nucleoside (cytidine) analog and the deoxy derivative of azacitidine, which has the formula:

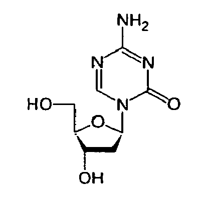

Both azacitidine and decitabine are used in the treatment of myelodysplastic syndrome.

Zebularine (CAS registry number of 3690-10-6) is another DMTI, which has the formula:

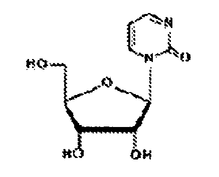

DMTIs cause many side effects, including, but are not limited to: low blood counts (where white, red blood cells and platelets may temporarily decrease, which may put the patient at increased risk for infection, anemia and/or bleeding, and may increase the need for blood or platelet transfusions), fatigue, fever, nausea, cough, petechiae (which can occur with low platelet count), constipation, diarrhea, hyperglycemia, headache, difficulty sleeping, swelling, low albumin, low magnesium, chills, low potassium, bruising, rash, low sodium, dizziness, generalized aches and pains, cardiac murmur, poor appetite, sore throat, abdominal pain, high bilirubin blood level, high potassium, mouth sores, drowsiness, abnormal liver function blood tests, confusion, anxiety, itching, and heartburn.

Due to these side effects, some patients who are on a DMTI would prefer to switch to another therapy, some of these patients cannot continue with the DMTI therapy and must switch to another agent for treating the MDS. It is also desirable for patients who do not respond or respond unsatisfactorily to a DMTI to switch to another MDS therapy. Other MDS patients who have been administered a DMTI for treating a tumor, may need an MDS therapy other than a DMTI. However, it has been unexpectedly found in a clinical trial that ezatiostat hydrochloride, a potential MDS agent, did not exhibit efficacy in patients who had prior exposure to DMTI treatment. As shown in Table 2, none of the evaluable patients in that study who had exposure to at least one DMTI prior to administration of ezatiostat hydrochloride responded to ezatiostat hydrochloride, whereas the response rate to ezatiostat hydrochloride in patients with no prior exposure to DMTI treatment is about 22 %. Such results limit the patient's choice in selecting an alternative therapy to replace the DMTI.

This unexpected problem can be solved by the surprising discovery that administration of lenalidomide prior to and/or currently with administration of ezatiostat hydrochloride to MDS patients who has prior exposure to a DMTI can retain the therapeutic effect of ezatiostat hydrochloride in treating an MDS. As shown in Table 2, the response rate to ezatiostat hydrochloride recovered to about 20 % in patients who were treated with both lenalidomide and a DMTI prior to administration of ezatiostat hydrochloride.

Accordingly, in one aspect, the invention is directed to a composition comprising ezatiostat or a salt thereof for use in treating a myelodysplastic syndrome in a patient who has been treated with a DNA methyltransferase inhibitor, wherein lenalidomide is administered to said patient prior to and/or concurrently with administration of said composition.

In some embodiments, the patient has been treated with at least one dosage of a DMTI. In some embodiments, the patient has been treated with the DMTI for at least 2 days, 3 days, 4 days, 5 days, or 6 days. In some embodiments, the patient has been treated with the DMTI for at least one week, two weeks or three weeks. In some embodiments, the patient has completed at least 1, 2, 3, 4, 5, or 6 treatment cycles. In some embodiments, the DMTI treatment is immediately prior to the administration of ezatiostat or a salt thereof. As used herein "immediately" means that the last DMTI dosage is administered no more than about one day prior to the first administration of ezatiostat or a salt thereof. In some embodiments, the DMTI treatment is less than 1 week prior to the administration of ezatiostat or a salt thereof. In some embodiments, the DMTI treatment is less than 1 month prior to the administration of ezatiostat or a salt thereof. In some embodiments, the DMTI treatment is less than 2 months, 6 months, or 12 months prior to the administration of ezatiostat or a salt thereof.

In some embodiments, the patient needs concurrent treatment with DMTI and ezatiostat or a salt thereof.

In some embodiments, lenalidomide is administered prior to administration of ezatiostat or a salt thereof. A typical lenalidomide treatment schedule involves a 28-day-cycle, during which lenalidomide is administered once a day every day for 21 days (3 weeks) followed by an interruption of 7 days (1 week) when no lenalidomide is administered. This 28-day-cycle can be repeated for a duration of up to 6 months. Lenalidomide capsules have four different strengths: 5 mg, 10 mg, 15 mg, and 25 mg.

In some embodiments, the patient is treated with at least one dosage of lenalidomide prior to administration of ezatiostat or a salt thereof. In some embodiments, the patient is treated with lenalidomide for at least 2 days, 3 days, 4 days, 5 days, or 6 days prior to administration of ezatiostat or a salt thereof. In some embodiments, the patient is treated with lenalidomide for at least one week, two weeks or three weeks prior to administration of ezatiostat or a salt thereof. In some embodiments, the patient is treated with lenalidomide for 1, 2, 3, 4, 5, or 6 treatment cycles prior to administration of ezatiostat or a salt thereof. In some embodiments, the patient is treated with the entire 6-month lenalidomide treatment regimen prior to administration of ezatiostat or a salt thereof.

In some embodiments, the patient is concurrently administered lenalidomide and ezatiostat or a salt thereof. In these cases, the patient may or may not be treated with lenalidomide prior to administration of ezatiostat or a salt thereof. When the patient is on prior lenalidomide treatment, the lenalidomide treatment may continue with administration of ezatiostat or a salt thereof at the same dosage and/or frequency, or at a reduced dosage and/or frequency, or treatment with lenalidomide may completely stop.

When administered concurrently, lenalidomide and ezatiostat or a salt thereof can be administered in any manner in which the pharmacological effects of both are manifested in the patient at the same time. Thus, concurrent administration of lenalidomide and ezatiostat or a salt thereof does not require that a single pharmaceutical composition, the same dosage form, or the same route of administration be used for the two agents. The two agents do not need to be administered at the same time or for a similar length of time. When administered by the same dosage form and the same route of administration, at substantially the same time, it could proceed by delivering both active ingredients simultaneously in a single novel pharmaceutical composition in accordance with the present invention. It is understood that in addition to the above, the invention contemplates that a concurrent administration may be the administration of a first and second pharmaceutical composition comprising lenalidomide and ezatiostat or a salt thereof, respectively. The term "concurrent" includes both simultaneous delivery as well as sequential delivery wherein each drug is administered separately in a manner that provides serum levels of both drugs in the patient at the same time.

In some embodiments of the invention, when administered concurrently with ezatiostat or a salt thereof, lenalidomide is administered in the typical 28-day-cycle as described above and may be given in any of the dosage strengths. In some embodiments, lenalidomide is administered at a reduced dosage and frequency, for example, lenalidomide may be administered once every other day, once every 3, 4, 5, or 6 days. Or it may be administered once a week or may be discontinued while treatment with ezatiostat or a salt thereof continues.

In some embodiments, the patient's prior exposure to DMTI is before administration of lenalidomide to the patient. In some embodiments, the patient's prior exposure to DMTI is after administration of lenalidomide to the patient. In some embodiments, the patient's prior exposure to DMTI is concurrent with administration of lenalidomide to the patient.

In some embodiments of the invention, ezatiostat or a salt thereof, for example, ezatiostat hydrochloride, is administered by a dosing regimen described in U.S. Patent Application No. 13/108,752, titled "COMPOSITIONS AND METHODS FOR TREATING MYELODYSPLASTIC SYNDROME," filed May 16, 2011 under the Attorney Docket No. 056274-4301.

Typically, ezatiostat or a salt thereof is administered in a therapeutically effective amount. In some embodiments of the invention, ezatiostat or a salt thereof is administered up to about 3.5 grams per day of ezatiostat hydrochloride, or an equivalent amount (in terms of ezatiostat content) of ezatiostat itself or another salt of ezatiostat. In a preferred embodiment, the dosing of ezatiostat or a salt thereof is a therapeutically effective amount of up to about 1.5 grams administered twice a day (b.i.d.).

In some embodiments, ezatiostat or a salt thereof is administered daily for at least 2 weeks. In some embodiments, ezatiostat or a salt thereof is administered daily for at least 3 weeks.

In one embodiment of the invention, ezatiostat or a salt thereof is administered in 1 gram dosages twice a day for three weeks followed by an interruption of one week where ezatiostat or a salt thereof is not administered. After the interruption, the regimen can be repeated as necessary. This regimen may be referred to as the "three-week regimen."

In another embodiment of the invention, ezatiostat or a salt thereof is administered in 1.5 gram dosages twice a day for two weeks followed by an interruption of one week where ezatiostat or a salt thereof is not administered. After the interruption, the regimen can be repeated as necessary. This regimen may be referred to as the "two-week regimen."

In another embodiment of the invention, the patient is treated continuously with a therapeutically effective amount of ezatiostat or a salt thereof of up to 3 grams per day preferably administered in up to 1.5 gram dosages twice a day. In this embodiment, ezatiostat or a salt thereof can be administered so long as the patient is in need of and can tolerate such treatment. It is contemplated that in this embodiment, the therapeutically effective amount of ezatiostat or a salt thereof may be less or more than that when there is an interruption in the treatment regimen. This regimen may be referred to as the "continuous regimen."

While twice a day administration is preferred, it is contemplated that once a day administration or 3 times a day administration could be employed. In the former case, once a day administration would assist in patient compliance; whereas in the latter case, smaller tablets could be used for those patients who have difficulty swallowing larger tablets. The amount of drug administered would be adjusted so that the total drug administered per day is a therapeutically effective amount.

The treatment with ezatiostat or a salt thereof may involve one or a combination of two or more of the dosing regimens described herein. The following are exemplifying dosing schedules of ezatiostat hydrochloride:
- 1.5 gram ezatiostat hydrochloride administered twice per day for 2 weeks for an aggregate total dosing of 42 grams followed by a week when no ezatiostat or a salt is administered;
- 1 gram ezatiostat hydrochloride administered twice per day for 3 weeks for an aggregate total dosing of 42 grams followed by a week when no ezatiostat or a salt is administered;
- 1 gram ezatiostat hydrochloride administered twice per day continuously until the attending clinician deems it appropriate for the patient to be withdrawn from administration;
- a therapeutically effective amount of up to 3 grams of ezatiostat hydrochloride per day administered in one, two, or three divided doses for 2 weeks followed by a week when no ezatiostat or a salt is administered;
- a therapeutically effective amount of up to 2 grams of ezatiostat hydrochloride per day administered in one, two, or three divided doses for 3 weeks followed by a week when no ezatiostat or a salt is administered; and/or
- a therapeutically effective amount of up to 2 grams of ezatiostat hydrochloride per day administered in one, two, or three divided doses continuously until the attending clinician deems it appropriate for the patient to be withdrawn from administration.

An equivalent amount of ezatiostat or another salt thereof (in terms of ezatiostat content) may replace ezatiostat hydrochloride in the above dosings.

When administration of ezatiostat or a salt thereof is twice a day, it is preferred that the interval between the first and second doses be from about 6 to 14 hours and preferably between about 8 and 14 hours.

In one embodiment, ezatiostat or a salt thereof, e.g., ezatiostat hydrochloride, can be administered intravenously as a lipid formulation such as those described in U.S. Patent No. 7,029,695.

In one embodiment, ezatiostat or a salt thereof can be administered orally. In another embodiment, ezatiostat or a salt thereof can be administered as a tablet formulation. Such a tablet formulation is disclosed in U.S. Patent Application No. 13/075,116, filed March 29, 2011, titled "TABLET FORMULATION OF EZATIOSTAT".

In another aspect, the invention provides a composition for treating a myelodysplastic syndrome in a patient who has been treated with a DNA methyltransferase inhibitor, which composition comprises lenalidomide, ezatiostat or a salt thereof, and optionally a pharmaceutically acceptable excipient. In some embodiments, the ezatiostat or the salt thereof and the lenalidomide together are in a therapeutically effective amount.

In some embodiments, the composition comprises about 5 mg, 10 mg, 15 mg or 25 mg of lenalidomide and about 200 mg, 300 mg, 400 mg, 500 mg, 600 mg, 700 mg, 800 mg, 900 mg, or 1000 mg ezatiostat or a salt thereof.

In one embodiment, lenalidomide may be added to a tablet formulation of ezatiostat or a salt thereof. Such a tablet formulation is disclosed in U.S. Patent Application No. 13/075,116, filed March 29, 2011, titled "TABLET FORMULATION OF EZATIOSTAT".

### 3. Kit

In still another aspect, the invention provides a kit for the treatment of MDS in a patient who has been treated with a DNA methyltransferase inhibitor, which kit comprises a first composition comprising lenalidomide and a second composition comprising ezatiostat or a salt thereof, including those described herein. In some embodiments, the ezatiostat or the salt thereof and the lenalidomide together are in a therapeutically effective amount.

In some embodiments, the kit further comprises a label with instructions to administer the first dose of lenalidomide 1 day, 2 days, 3 days, 4 days, 5 days, 6 days before the first administration of ezatiostat or a salt thereof. In some embodiments, the kit further comprises a label with instructions to administer lenalidomide 1 week, 2 weeks, 3 weeks, 4 weeks, 5 weeks, or 6 weeks before administration of ezatiostat or a salt thereof. In some embodiments, the kit further comprises a label with instructions to administer lenalidomide concurrently with ezatiostat or a salt thereof. In some embodiments, the kit further comprises a label with instructions to administer lenalidomide and ezatiostat or a salt thereof according to any of the dosing schedules described herein.

### Examples

The present invention is further defined by reference to the following examples.

### Ezatiostat hydrochloride tablets in patients with an International Prognostic System Score (IPSS) low to intermediate-1 risk myelodysplastic syndrome

Eighty-seven patients were randomized and treated at 23 investigational sites. After initial dose ranging in 14 patients, two dose levels were selected for further study. Subsequently, 37 patients were treated at 3 grams daily for two weeks followed by a one week rest period, and 36 patients were treated at 2 grams daily for three weeks followed by a one week rest period. The data on these 73 patients was pooled for this preliminary analysis.

The median age was 72 years, with a patient population distribution of IPSS low risk (23 patients, 32 %) and intermediate-1 risk (50 patients, 68 %). Patients had received a median of three prior MDS therapies including, 34 patients (47 %) with prior Revlimid® (lenalidomide) and 28 patients (38 %) with prior DNA methyltransferase inhibitors (DMTI) [azacitidine, decitabine].

At the time of preliminary analysis, 8 patients remained on treatment for continuing clinical benefit. The overall Hematologic Improvement - Erythroid (HI-E) rate was 22 %, 13 of 60 evaluable patients (95 % Cl, 12.1-34.2). The median duration of HI-E response was 46 weeks (range 2-51). The median hemoglobin level increased by 2.0 gram/dL in responders. Eleven of 38 red blood cell (RBC) transfusion-dependent patients (29 %) had clinically significant RBC transfusion reductions (reduction of 4U/8 weeks, IWG 2006) with 4 patients (11 %) achieving RBC transfusion independence and 3 patients continuing on treatment. In addition, one patient continued in complete remission for more than 12 months following discontinuation of therapy (Quddus, et al., J. Hem. and Onc. Apr. 2010, 3:15).

Telintra® continues to demonstrate multilineage hematologic improvement. There was a 15 % Hematologic Improvement - Neutrophil (HI-N) rate observed in 3 of 20 patients (95 % Cl, 3.2-37.9), and the bilineage Hl rate (Hl-E and Hl-N) was 11 %, 2 of 19 patients (95 % Cl, 1.3-33.1).

There were three cytogenetic complete responses, one in a patient with 45X,-Y[4], 46, XY [16] abnormal cytogenetics that converted to normal after four cycles of therapy. Of the four patients enrolled in the study with del 5q minus, two had a complete cytogenetic response, including one who had failed prior Revlimid® therapy.

A planned logistic regression analysis was used to evaluate all known prognostic characteristics in order to define those patients who had an increased likelihood of HI-E response to Telintra®. Prior DMTI treatment predicts a five-fold decrease in the odds for a HI-E response to Telintra® (p=0.023). Prior Revlimid® treatment was observed to enhance HI-E response to Telintra®.
- There was a 40 % HI-E rate (6 of 15 patients, 95 % Cl, 16.3 %-67.7 %) in patients who had prior Revlimid® treatment, but no prior DMTI treatment. Within this patient group, five of 11 patients (45 %) achieved significant RBC transfusion reduction with three of those patients (27 %) achieving transfusion independence.
- There was a 26% HI-E rate (6 of 23 patients, 95 % Cl, 10.2 %-48.4 %) in patients who had no prior Revlimid® treatment and no prior DMTI treatment. Within this group, five of 11 patients (45%) achieved significant RBC transfusion reduction.
- There was a 0 % HI-E rate (0 of 17 patients, 95 % Cl, 0 %-19.5 %) in patients who had no prior Revlimid® treatment but who had received prior DMTI treatment.

More than 403 cycles of Telintra® therapy have been administered. The safety data is based on all patients treated. The most common non-hematologic adverse events (AEs) were Grade 1 and 2 gastrointestinal (Gl) respectively, nausea (45%, 16%), diarrhea (25%, 7%) and vomiting (30%, 12%). Grade 3 events were uncommon: nausea (1%), diarrhea (3%) and vomiting (2%). Prior DMTI treatment was associated with an increased incidence of Gl AEs.

Telintra® treatment may result in clinically significant hematologic improvement in patients with MDS and may offer an alternative to RBC transfusions. These results are consistent with levels of efficacy observed in prior studies with Telintra®, the first GST P1-1 enzyme inhibitor tested in MDS patients.

Tables 1 and 2 summarize the results of this clinical study.

**Table 1**

| Hematological Improvement-Erythroid (HI-E): Time to Response and Duration of Response Starting Telintra® Dose of 3,000 mg/day (1.5 g b.i.d.) or 2,000 mg/day (1 g b.i.d.) (Efficacy Evaluable Population) | | |
|---|---|---|
| | Telintra® Dosing Schedule | |
| | 1.5 g b.i.d. 2 weeks on & 1 week off (N=29) | 1 g b.i.d. 3 weeks on & 1 week off (N=31) |

| Time to HI-E Response (Weeks) [1] | | |
|---|---|---|
| N | 7 | 6 |
| Mean | 8.4 (0.72) | 8.9 (1.29) |
| Median | 8.1 | 8.4 |
| Min, Max | 8.0, 10.0 | 8.0, 11.3 |

| Duration of HI-E Response (Weeks) [2] | | |
|---|---|---|
| # Event | 5 (71.4 %) | 2 (33.3%) |
| # Censored | 2 (28.6 %) | 4 (66.7%) |
| Median (95 % Cl) | 18.4 (3.1-51.0) | 46.1 (10.0-46.1) |
| Min, Max | 1.9-51.0 | 2.4-46.1 |

| | | |
|---|---|---|
| [1] Days from date of first dose of study medication to the date of first documentation of response plus one divided by 7. [2] Total number of days of where response is seen divided by 7. | | |

**Table 2**

| Hematological Improvement - Erythroid (HI-E): by Revlimid^{®} and DNA methyltransferase inhibitors failure status (Efficacy evaluable population) | | | | |
|---|---|---|---|---|
| Revlimid® | DMTI[2] | HI-E [1] Statistics | | |
| | | N | Response (n) | Response Rate (95 % Confidence Interval) |
| Yes | Yes | 15 | 3 | 20 % (4.3 % - 48.1 %) |
| No | Yes | 15 | 0 | 0.0 % (0.0 % - 21.8 %) |
| No | No | 27 | 6 | 22.2 % (8.6 % - 42.3 %) |

| | | | | |
|---|---|---|---|---|
| [1] RBC transfusion reduction from baseline => 4 units per eight weeks; or patient with symptomatic anemia not transfusion dependent with hemoglobin < 11 g/dL prior to treatment, achieving a hemoglobin increase by >= 1.5 g/dL sustained for a period of eight weeks. [2] Including azacitidine or decitabine. | | | | |

Table 2 shows that: (1) when Telintra® was given to patients with no prior treatment of either Revlimid® or a DMTI, the response rate to Telintra® was about 22 %; (2) when Telintra® was given to patients with prior DMTI treatment, none of the patients responded to Telintra®; and (3) the response rate to Telintra® was about 20 % for patients who were treated with both Revlimid® and a DMTI prior to treatment with Telintra®.

## Claims

1. A composition comprising ezatiostat or a salt thereof for use in treating a myelodysplastic syndrome in a patient who has been treated with a DNA methyltransferase inhibitor, wherein lenalidomide is administered to said patient prior to and/or concurrently with administration of said composition.

2. The composition for use according to claim 1, wherein the lenalidomide is administered prior to administration of the composition.

3. The composition for use according to claim 1, wherein the lenalidomide is administered concurrently with administration of the composition.

4. The composition for use according to any one of claims 1 to 3, which is administered daily for at least 2 weeks.

5. The composition for use according to any one of claims 1 to 4, which is administered orally.

6. The composition for use according to any one of claims 1 to 5, wherein the ezatiostat or salt thereof is ezatiostat hydrochloride.

7. The composition for use according to claim 6, wherein the ezatiostat hydrochloride is administered according to a treatment schedule comprising administration of a daily dosage of 2 grams for three weeks followed by a one-week interruption wherein no ezatiostat or a salt thereof is administered.

8. The composition for use according to claim 6, wherein the ezatiostat hydrochloride is administered according to a treatment schedule comprising administration of a daily dosage of 3 grams for two weeks followed by a one-week interruption wherein no ezatiostat or a salt thereof is administered.

9. A composition for use in treating a myelodysplastic syndrome in a patient who has been treated with a DNA methyltransferase inhibitor, which composition comprises lenalidomide, ezatiostat or a salt thereof , and optionally a pharmaceutically acceptable excipient.

10. The composition for use according to claim 9, wherein the ezatiostat or the salt thereof and the lenalidomide together are in a therapeutically effective amount.

11. A kit for use in treating a myelodysplastic syndrome in a patient who has been treated with a DNA methyltransferase inhibitor, which kit comprises a first composition comprising lenalidomide and a second composition comprising ezatiostat or a salt thereof.

12. The kit for use according to claim 11, wherein the ezatiostat or the salt thereof and the lenalidomide together are in a therapeutically effective amount.

## Patentansprüche

1. Zusammensetzung, die Ezatiostat oder ein Salz davon umfasst, zur Verwendung bei der Behandlung eines myelodysplastischen Syndroms bei einem Patienten, der mit einem DNA-Methyltransferase-Inhibitor behandelt wurde, wobei dem genannten Patienten vor und/oder gleichzeitig mit der Verabreichung der genannten Zusammensetzung Lenalidomid verabreicht wird.

2. Zusammensetzung zur Verwendung nach Anspruch 1, wobei das Lenalidomid vor der Verabreichung der Zusammensetzung verabreicht wird.

3. Zusammensetzung zur Verwendung nach Anspruch 1, wobei das Lenalidomid gleichzeitig mit der Verabreichung der Zusammensetzung verabreicht wird.

4. Zusammensetzung zur Verwendung nach irgendeinem der Ansprüche 1 bis 3, die wenigstens 2 Wochen lang täglich verabreicht wird.

5. Zusammensetzung zur Verwendung nach irgendeinem der Ansprüche 1 bis 4, die oral verabreicht wird.

6. Zusammensetzung zur Verwendung nach irgendeinem der Ansprüche 1 bis 5, wobei das Ezatiostat oder Salz davon Ezatiostat-Hydrochlorid ist.

7. Zusammensetzung zur Verwendung nach Anspruch 6, wobei das Ezatiostat-Hydrochlorid gemäß einem Behandlungsplan verabreicht wird, der die Verabreichung einer täglichen Dosis von 2 Gramm über drei Wochen beinhaltet, gefolgt von einer einwöchigen Unterbrechung, bei der kein Ezatiostat oder Salz davon verabreicht wird.

8. Zusammensetzung zur Verwendung nach Anspruch 6, wobei das Ezatiostat-Hydrochlorid gemäß einem Behandlungsplan verabreicht wird, der die Verabreichung einer täglichen Dosis von 3 Gramm über zwei Wochen beinhaltet, gefolgt von einer einwöchigen Unterbrechung, bei der kein Ezatiostat oder Salz davon verabreicht wird.

9. Zusammensetzung zur Verwendung bei der Behandlung eines myelodysplastischen Syndroms bei einem Patienten, der mit einem DNA-Methyltransferase-Inhibitor behandelt wurde, wobei die Zusammensetzung Lenalidomid, Ezatiostat oder ein Salz davon und optional einen pharmazeutisch akzeptablen Exzipienten umfasst.

10. Zusammensetzung zur Verwendung nach Anspruch 9, wobei das Ezatiostat oder Salz davon und das Lenalidomid zusammen in einer therapeutisch effektiven Menge vorliegen.

11. Kit zur Verwendung bei der Behandlung eines myelodysplastischen Syndroms bei einem Patienten, der mit einem DNA-Methyltransferase-Inhibitor behandelt wurde, wobei der Kit eine erste Zusammensetzung, die Lenalidomid umfasst, und eine zweite Zusammensetzung umfasst, die Ezatiostat oder ein Salz davon umfasst.

12. Kit zur Verwendung nach Anspruch 11, wobei das Ezatiostat oder Salz davon und das Lenalidomid zusammen in einer therapeutisch effektiven Menge vorliegen.

## Revendications

1. Composition comprenant l'ezatiostat ou un sel de celui-ci pour une utilisation dans le traitement d'un syndrome myélodysplasique chez un patient qui a été traité par un inhibiteur de l'ADN-méthyltransférase, où le lénalidomide est administré au dit patient avant et/ou pendant l'administration de ladite composition.

2. Composition pour une utilisation selon la revendication 1, où le lénalidomide est administré avant l'administration de ladite composition.

3. Composition pour une utilisation selon la revendication 1, où le lénalidomide est administré simultanément à l'administration de ladite composition.

4. Composition pour une utilisation selon l'une quelconque des revendications 1 à 3, qui est administrée quotidiennement pendant au moins 2 semaines.

5. Composition pour une utilisation selon l'une quelconque des revendications 1 à 4, qui est administrée par voie orale.

6. Composition pour une utilisation selon l'une quelconque des revendications 1 à 5, où l'ezatiostat ou le sel de celui-ci est le chlorhydrate d'ezatiostat.

7. Composition pour une utilisation selon la revendication 6, où le chlorhydrate d'ezatiostat est administré conformément à un schéma posologique qui comprend une période de traitement à une dose quotidienne de 2 grammes pendant trois semaines suivie par une interruption d'une semaine sans administrations d'ezatiostat ou d'un sel de celui-ci.

8. Composition pour une utilisation selon la revendication 6, où le chlorhydrate d'ezatiostat est administré conformément à un schéma posologique qui comprend une période de traitement à une dose quotidienne de 3 grammes pendant deux semaines suivie par une interruption d'une semaine sans administrations d'ezatiostat ou d'un sel de celui-ci.

9. Composition pour une utilisation dans le traitement d'un syndrome myélodysplasique chez un patient qui a été traité par un inhibiteur de l'ADN-méthyltransférase, ladite composition comprenant le lénalidomide, l'ezatiostat ou un sel de celui-ci et, en option, un excipient pharmaceutiquement acceptable.

10. Composition pour une utilisation selon la revendication 9, où l'ezatiostat ou le sel de celui-ci et le lénalidomide sont utilisés ensemble à une quantité thérapeutiquement efficace.

11. Kit pour une utilisation dans le traitement d'un syndrome myélodysplasique chez un patient qui a été traité par un inhibiteur de l'ADN-méthyltransférase, ledit kit comprenant une première composition comprenant le lénalidomide et une seconde composition comprenant l'ezatiostat ou un sel de celui-ci.

12. Kit pour une utilisation selon la revendication 11, où l'ezatiostat ou le sel de celui-ci et le lénalidomide sont utilisés ensemble à une quantité thérapeutiquement efficace.
